# EUROPEAN PATENT SPECIFICATION

(11) **EP 2 625 145 B1**
(45) Date of publication and mention of the grant of the patent: **05.06.2019**
(21) Application number: 11824058.9
(22) Date of filing: 07.09.2011
(51) Int. Cl.: C02F 1/32, B01D 53/86

(54) **ENHANCED PHOTO-CATALYTIC CELLS**
VERBESSERTE PHOTOKATALYTISCHE ZELLEN
CELLULES PHOTO-CATALYTIQUES AMÉLIORÉES

(30) Priority: 25.05.2011 US 201113115546; 07.09.2010 US 380462 P
(43) Date of publication of application: 14.08.2013
(73) Proprietor: Puradigm, LLC, Las Vegas, NV 89101 (US)
(72) Inventor: TUPMAN, David E., Anna Texas 75409 (US)
(74) Representative: Kuhnen & Wacker Patent- und Rechtsanwaltsbüro PartG mbB
(86) International application number: PCT/US2011/050666
(87) International publication number: WO 2012/033818

(56) References cited:
- EP-A1- 1 772 187
- WO-A1-2005/030371
- WO-A1-2008/144519
- WO-A2-2005/091787
- US-A1- 2004 166 037
- US-A1- 2004 248 075
- US-A1- 2005 063 881
- US-B1- 6 500 387
- US-B1- 6 752 957

## Description

### RELATED APPLICATIONS

### BACKGROUND OF THE INVENTION

The present invention relates to an apparatus for producing an enhanced ionized cloud of bactericidal molecules.

Photo-catalytic cells may be employed to produce bactericidal molecules in air flow passing through the cells. The cells may be positioned to ionize air that may then be directed into an enclosed space or room. Emerging molecules from the cells may have a bactericidal effect on various bacteria, molds or viruses which may be airborne in the room or may be on surfaces of walls or objects in the room.

Typically, such cells may be constructed with a "target material" (or coated surface(s) surrounding a broad spectrum ultraviolet (UV) emitter. This combination can produce an ionized cloud of bactericidal molecules. The target may be coated with titanium dioxide as well as a few other proprietary trace elements. As air passes through or onto the target, UV energy striking the titanium dioxide may result in a catalytic reaction that may produce the desired cloud of bactericidal molecules within the airflow. These molecules, upon contact with any bacteria, mold or virus, may kill them.

Effectiveness of such photo-catalytic cells may be dependent on the concentration of the bactericidal molecules which may be produced by the cells. The bactericide concentration level may be dependent on the degree to which UV energy is applied to the titanium dioxide of the honeycomb mesh.

An apparatus for ionizing air according the preamble of claim 1 is known from US 2004/166037 A1. The apparatus comprises a primary particulate filter for removal of at least a portion of particulate matter entrained in the air flow, at least one ultraviolet lamp, and a permeable reaction filter. The ultraviolet lamp is positioned downstream of the primary particulate filter and the permeable reaction filter is positioned downstream of the ultraviolet light. The permeable reaction filter has a substrate member and a plurality of titanium dioxide particles bonded to portions of the substrate member to form a photo-catalytic oxidizer layer disposed thereon a portion of the substrate member. The ultraviolet lamp irradiates at least a portion of the permeable reaction layer so that at least a portion of contaminants entrained in the air flow.

WO 20087144519 A1 teaches an apparatus for making a photocatalytic oxidation substrate by powder coating a photocatalyst onto a metal substrate.

From EP 1 772 187 A1 a surface light-emitting device is known wherein a catalytic reaction can be efficiently performed without the use of an ultraviolet lamp, an ultraviolet LED, or another such external ultraviolet light source, even in the case of a highly UV-absorbing contaminated fluid that cannot be treated with an external light source.

WO 2005/030371 A1 shows lamp including a reflective portion is utilized in a fluid purification system to maximize the light delivery to a photocatalytic coating that oxidizes gaseous contaminants that adsorb onto the surface to form carbon dioxide, water, and other substances. An ultraviolet light source positioned proximate to the honeycomb activates the titanium dioxide coating. In one example, the reflective portion is a reflective coating. Light directed out of the non-reflective portion of the lamp travels towards the honeycomb and absorbs onto the photocatalytic coating. Light directed towards the reflective portion on the lamp is reflected off the surface of the reflective portion and passes through the non-reflective portion of the lamp to also absorb onto the photocatalytic coating. The reflective portion reflects light towards the honeycomb that would otherwise be misdirected away from the honeycomb, increasing efficiency of the fluid purification system.

The apparatus of WO 2005/091787 A2 includes an ultraviolet light source located between two titanium dioxide coated honeycombs. When photons of ultraviolet light are absorbed by the titanium dioxide coating, reactive hydroxyl radicals are formed that attack and oxidize contaminants in the air to water, carbon dioxide, and other substances. A shield is positioned outside each of the honeycombs to minimize the direct leakage of ultraviolet light from the module. The height of each shield depends on the distance between each shield and the ultraviolet light source and the maximum angle that ultraviolet light can pass through the honeycomb without any reflection on the surface of the honeycomb.; Any ultraviolet light that directly leaks from the honeycomb contacts the shield and reflects towards honeycomb, minimizing the direct leakage of ultraviolet light from the module and increasing the photocatalytic rate of the contaminants.

The apparatus shown in US 2005/063881 A1 includes a treatment chamber provided with an inlet and an outlet and defining an interior surface. A radiation source is provided within the treatment chamber. A photocatalytic reactor purifying the fluid upon receiving radiation emitted from the radiation source is also provided within the purification system. The interior surface of the treatment chamber is substantially reflective for radiation emitted by the radiation source.

As can be seen, there is a need for a system in which a higher proportion of UV energy from a UV emitter (in such a photo-catalytic cell) can be caused to impinge upon the titanium dioxide within the cell.

### SUMMARY OF THE INVENTION

An apparatus comprising the features of claim 1 and a method according to claim 9 solve this problem. Further improvements are subject to the dependent claims.

These and other features, aspects and advantages of the present invention will become better understood with reference to the following drawings, description and claims.

### BRIEF DESCRIPTION OF THE DRAWINGS

Figure 1 is a perspective view of a typical photo-catalytic cell in accordance with an embodiment of the invention in which a typical "honeycomb matrix" is shown as the "target";
Figure 2 is a side elevation view of the photo-catalytic cell of Figure 1;
Figure 3 is a cross sectional view of the photo-catalytic cell of Figure 2 taken along the line 3-3; and

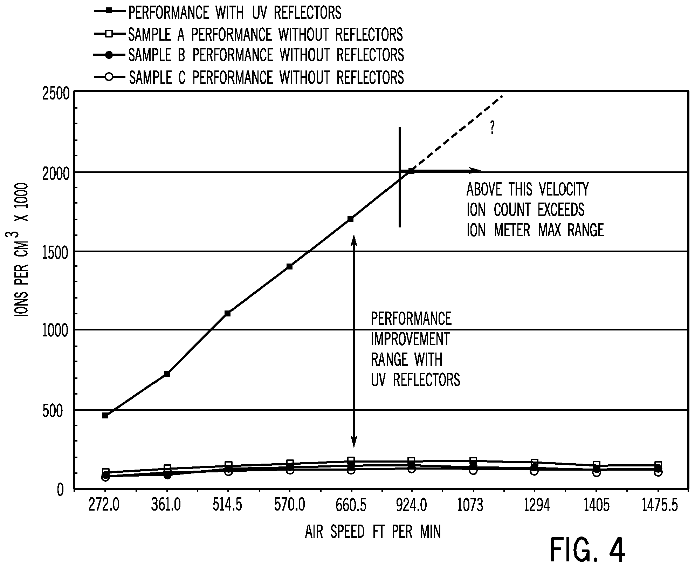

Figure 4 is a comparison graph showing a difference in performance of the photo-catalytic cell of Figure 1 with and without use of UV reflectors in accordance with the invention.

### DETAILED DESCRIPTION OF THE INVENTION

The following detailed description is of the best currently contemplated modes of carrying out exemplary embodiments of the invention. The description is not to be taken in a limiting sense, but is made merely for the purpose of illustrating the general principles of the invention, since the scope of the invention is best defined by the appended claims.

Various inventive features are described below that can each be used independently of one another or in combination with other features.

Broadly, embodiments of the present invention generally provide photo-catalytic cells in which reflectors may be positioned to reflect UV energy and increase a proportion of emitted UV energy that strikes titanium dioxide in the cell at high incident angles.

Referring now to the Figures, it may be seen that an exemplary embodiment of a photo-catalytic cell 10 may comprise an electronics box 12; a light pipe indicator 14; a power cord 16; a chamber 18; honeycomb targets 20; UV reflectors 22-1, 22-2 and 22-3; and a UV emitter or lamp 24. The honeycomb targets 20 may be coated with titanium dioxide.

In operation, air may pass across the honeycomb targets 20 while UV energy may be applied to the target 20 by the lamp 24. A photo-catalytic reaction may take place in the presence of the UV energy. The reaction may produce bactericidal molecules in the air.

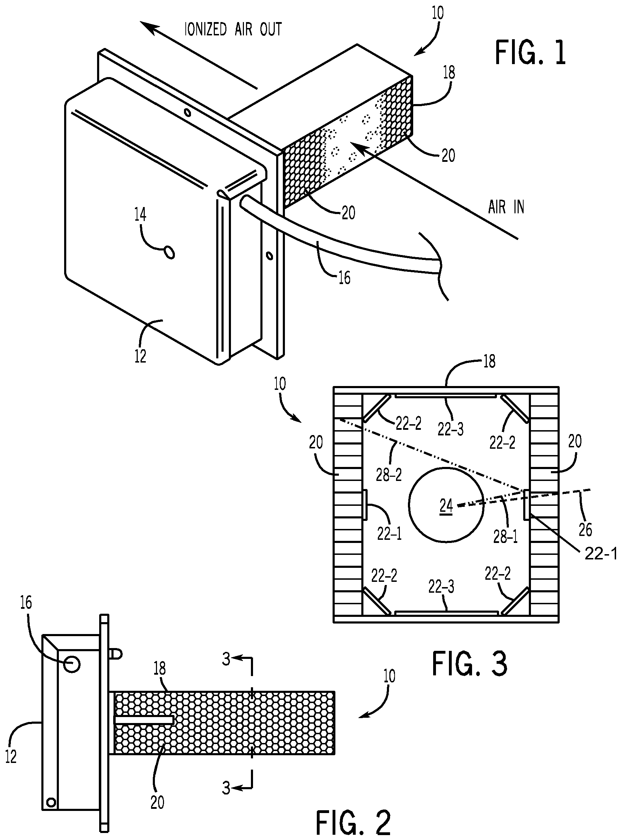

Referring now particularly to Figure 3, the efficacy of the UV reflectors 22-1 may be illustrated. If the reflector 22-1 were not present, an emitted ray 26 might pass through the honeycomb target 20 without impinging on the titanium dioxide. However, when one of the reflectors 22-1 is present, an illustrative emitted ray 28-1 of UV energy may impinge on the UV reflectors 22-1. The ray 28-1 may be reflected to become a reflected ray 28-2. It may be seen that the reflected ray 28-2 may impinge on a surface of the honeycomb target 20. It may be seen that a hypothetical unreflected ray 26, which might follow a path parallel to that of the ray 28-1, might pass through the honeycomb target 20 without impinging on the target 20. Thus, presence of the reflector 22-1 in the path of the ray 28-1 may result in avoidance of loss of the UV energy from the ray 28-1. The reflectors 22-1 may be relatively small as compared to the size of the honeycomb target 20. The small size (about 10% of the size of the target 20) may allow for minimal air flow obstruction. In spite of their relatively small size, the reflectors 22-1 may be effective because they may reflect virtually all of the (normally lost) UV energy that is emitted in a direction that is almost orthogonal (i.e., within ± 5° of orthogonality) to the outer vertical plane of the honeycomb target 20. Hence, UV energy would pass thru the honeycomb target without touching the TiO2 surface. But by "reflecting" the UV rays onto the "opposite side" target matrix - - that energy could be captured and utilized so as to add to the total ion count within the desired cloud of ionized molecules. In other words, the number of ions created by any incoming UV ray is proportional to the sine of the incident angle (Theta) between the UV ray path and the TiO2 surface that a given ray is impacting.

| | | |
|---|---|---|
| At theta = 90 deg | Sine (90) = 1 | Maximum energy gathered |
| At theta = 0 deg | Sine (0) = 0 | Minimum energy gathered |

Reflectors 22-3 may be interposed between the lamp 24 and walls of the chamber 18. UV energy striking the reflectors 22-3 may be reflected onto the honeycomb target 20. Thus presence of the reflectors 22-3 may result in avoidance of loss of UV energy that might otherwise be absorbed or diffused by walls of the chamber 18. Similarly, reflectors 22-2 may be placed in corners of the chamber 18 to reflect UV energy onto the honeycomb target 20.

The reflectors 22-1, 22-2 and/or 22-3 may be constructed from material that is effective for reflection of energy with a wavelength in the UV range (i.e., about 184 nanometers [nm] to about 255 nm). While soft metals such as gold and silver surfaces may be effective reflectors for visible light, their large grain size may make them less suitable than metallic surfaces with a small grain size (i.e., hard metals). Thus, hard metals such as chromium and stainless steel and other metals that do not readily oxidize may be effective UV reflectors and may be particularly effective for use as UV reflectors in the photo-catalytic cell 10. Material with a UV reflectivity of about 90% or higher may be suitable for use in the reflectors 22-1, 22-1 and 22-3. Lower reflectively produces lower effectiveness. To achieve the level of reflection required, it may be necessary to "micro-polish or buff" a selected materials reflective surface to achieve the specifications defined in para 22] - 24] below.

Advantageously, reflecting surfaces of the reflectors 22 should be electrically conductive. Specifically, outer surface coatings (added for oxidation protection) like glass, clear plastics, clear anodization (i.e. non-conductive) may diminish (considerably) any performance enhancement of the photo-catalytic cell 10.

Also it is important that reflecting surfaces of the UV reflector 22 produce surface specular reflection. (Specular reflection being a "mirror-like reflection" of light - - in which a single incoming light ray is reflected into a single outgoing direction) Specular reflection is distinct from "diffuse" reflection where an incoming light ray is reflected into a broad range of directions. Diffuse reflection may diminish performance enhancement of the photo-catalytic cell 10.

In an exemplary embodiment of the photo-catalytic cell 10, the reflectors 22-1, 22-2 and 22-3 may be chromium-plated plastic. Chromium-plated plastic may be a desirably low cost material with a desirably high degree of reflectivity for UV energy. So called "soft chrome" such as the plating used to produce a mirror-like finish that is seen on automobile chromed surfaces may be advantageously employed.

It may be noted that there may be other cell shape designs which are not rectangular. For example, the cell 10 may be circular, tubular, or may have an otherwise complex shape. For these non-rectangular shaped cells, an optimum reflector design may be curved or otherwise non-flat in shape.

## Claims

1. An apparatus for ionizing air, the apparatus comprising:
an UV emitter (24);
a first wall and a second wall opposite to the first wall;
a first target (20), the first target (20) including:
an inner face arranged to receive ultra-violet ("UV") energy from the UV emitter (24),
an outer face,
a plurality of passages between the inner face and the outer face, and
a photo-catalytic coating on the plurality of passages;
a first specular reflector (22-3) interposed between the UV emitter (24) and first
wall;
a second specular reflector (22-3) interposed between the UV emitter (24) and
second wall;
**characterized in**:
a second target (20) opposite the first target (20), the second target (20) including:
an inner face arranged to receive ultra-violet ("UV") energy from the UV emitter (24),
an outer face,
a plurality of passages between the inner face and the outer face, and
a photo-catalytic coating on the plurality of passages;
and
wherein the targets (20) comprise UV reflectors (22-1) configured to prevent an emitted UV ray (26) to pass through the targets (20).

2. The apparatus of claim 1, wherein a reflecting surface of the first and second reflectors (22-3) are electrically conductive.

3. The apparatus of claim 1, wherein the first and second reflectors (22-3) comprise micro-polished stainless steel.

4. The apparatus of claim 1, wherein the first and second reflectors (22-3) comprise a material that does not readily oxidize.

5. The apparatus of claim 1, wherein the first and second reflectors (22-3) comprise a material having a UV reflectivity of about 90% or greater at UV wavelengths of 185 nm and 254 nm.

6. The apparatus of claim 1 wherein the first target (20) and/or the second target (20) comprise a honeycomb matrix.

7. The apparatus of claim 1, further comprising:
a third reflector (22-2) placed in a corner between the first target (20) and the first reflector (22-3);
a fourth reflector (22-2) placed in a corner between the second target (20) and the first reflector (22-3);
a fifth reflector (22-2) placed in a corner between the first target (20) and the second reflector (22-3); and
a sixth reflector (22-2) placed in a corner between the first target (20) and the second reflector (22-3).

8. The apparatus of claim 1, further comprising a seventh reflector (22-1) and/or an eight reflector (22-1) on the inner face of the first target (20) or second target (20) and configured to reflect UV energy that is emitted from the UV emitter in a direction that is almost orthogonal to the inner surface of the first target (20) or second target (20), wherein the seventh reflector and/or the eight reflector (22-1) is a specular UV reflector.

9. A method for ionizing air, the method comprising:
emitting, by an ultra-violet ("UV") emitter (24), UV energy towards a first reflector (22-3), a second reflector (22-3) opposite to the first reflector (22-3), a first target (20) and a second target (20) opposite to the first target (20);
reflecting, by the first reflector (22-3) and the second reflector (22-3), UV energy towards the first target (20) and the second target (20), wherein the first and second reflectors are specular UV reflectors;
receiving, at a plurality of passages through the first and second targets (20), UV energy directly from the UV emitter (24) and UV energy reflected from the first and second reflectors (22-3);
responsively generating ions at a photo-catalytic coating on the plurality of passages of the first and second targets (20); and
passing an airflow through the plurality of passages of the first and second targets (20) to carry the ions away from the first and second targets (20),
**characterised by**
preventing an emitted UV ray (26) to pass through the targets (20) by UV reflectors (22-1).

10. The method of claim 9, wherein the step of responsively generating ions further comprises responsively generating more ions at the photo-catalytic coating in response to receiving UV energy from the first and second reflectors (22-3) than in response to receiving UV energy directly from the UV emitter (24).

11. The method of claim 9, wherein the first and second reflectors (22-3) comprise a material having a UV reflectivity of about 90% or greater at UV wavelengths of 185 nm and 254 nm.

## Patentansprüche

1. Vorrichtung zum Ionisieren von Luft, wobei die Vorrichtung aufweist:
einen UV-Emitter (24);
eine erste Wand und eine zweite Wand, die der ersten Wand gegenüberliegt;
ein erstes Ziel (20), wobei das erste Ziel (20) beinhaltet:
eine Innenfläche, die so angeordnet ist, dass sie ultraviolette ("UV") Energie von dem UV-Emitter (24) empfängt,
eine Außenfläche,
eine Mehrzahl an Durchlässen zwischen der Innenfläche und der Außenfläche, und
eine photokatalytische Beschichtung auf der Mehrzahl an Durchlässen;
einen ersten Spiegelreflektor (22-3), der sich zwischen dem UV-Emitter (24) und der ersten Wand befindet;
einen zweiten Spiegelreflektor (22-3), der sich zwischen dem UV-Emitter (24) und der zweiten Wand befindet;
**gekennzeichnet durch**:
ein zweites Ziel (20), das dem ersten Ziel (20) gegenüberliegt, wobei das zweite Ziel (20) beinhaltet:
eine Innenfläche, die so angeordnet ist, dass sie ultraviolette ("UV") Energie von dem UV-Emitter (24) empfängt,
eine Außenfläche,
eine Mehrzahl an Durchlässen zwischen der Innenfläche und der Außenfläche, und
eine photokatalytische Beschichtung auf der Mehrzahl an Durchlässen; und
wobei die Ziele (20) UV-Reflektoren (22-1) aufweisen, die so konfiguriert sind, dass es einem emittierten UV-Strahl (26) unmöglich ist, durch die Ziele (20) hindurch zu verlaufen.

2. Vorrichtung nach Anspruch 1, wobei eine reflektierende Oberfläche der ersten und zweiten Reflektoren (22-3) elektrisch leitend ist.

3. Vorrichtung nach Anspruch 1, wobei der erste und der zweite Reflektor (22-3) mikropolierten Edelstahl aufweisen.

4. Vorrichtung nach Anspruch 1, wobei der erste und der zweite Reflektor (22-3) ein Material aufweisen, das nicht leicht oxidiert.

5. Vorrichtung nach Anspruch 1, wobei der erste und der zweite Reflektor (22-3) ein Material aufweisen, das ein UV-Reflexionsvermögen von ungefähr 90% oder mehr bei UV-Wellenlängen von 185 nm und 254 nm aufweist.

6. Vorrichtung nach Anspruch 1, wobei das erste Ziel (20) und/oder das zweite Ziel (20) eine Honigwabenmatrix aufweisen.

7. Vorrichtung nach Anspruch 1, ferner aufweisend:
einen dritten Reflektor (22-2), der in einer Ecke zwischen dem ersten Ziel (20) und dem ersten Reflektor (22-3) platziert ist;
einen vierten Reflektor (22-2), der in einer Ecke zwischen dem zweiten Ziel (20) und dem ersten Reflektor (22-3) platziert ist;
einen fünften Reflektor (22-2), der in einer Ecke zwischen dem ersten Ziel (20) und dem zweiten Reflektor (22-3) platziert ist; und
einen sechsten Reflektor (22-2), der in einer Ecke zwischen dem ersten Ziel (20) und dem zweiten Reflektor (22-3) platziert ist.

8. Vorrichtung nach Anspruch 1, ferner aufweisend einen siebenten Reflektor (22-1) und/oder einen achten Reflektor (22-1) auf der Innenfläche des ersten Ziels (20) oder zweiten Ziels (20), die so konfiguriert sind, dass sie die UV-Energie, die von dem UV-Emitter emittiert wird, in eine Richtung reflektieren, die zu der Innenoberfläche des ersten Ziels (20) oder zweiten Ziels (20) beinahe rechtwinklig ist, wobei der siebente Reflektor und/oder der achte Reflektor (22-1) ein UV-Spiegelreflektor ist.

9. Verfahren zum Ionisieren von Luft, wobei das Verfahren aufweist:
Emittieren, durch einen Ultraviolett-("UV")-Emitter (24), von UV-Energie in Richtung eines ersten Reflektors (22-3), eines zweiten Reflektors (22-3), der dem ersten Reflektor (22-3) gegenüberliegt, eines ersten Ziels (20) und eines zweiten Ziels (20), das dem ersten Ziel (20) gegenüberliegt;
Reflektieren, durch den ersten Reflektor (22-3) und den zweiten Reflektor (22-3), von UV-Energie in Richtung des ersten Ziels (20) und des zweiten Ziels (20), wobei der erste und der zweite Reflektor UV-Spiegelreflektoren sind;
Empfangen, an einer Mehrzahl an Durchlässen durch das erste und zweite Ziel (20), von UV-Energie direkt von dem UV-Emitter (24) und von UV-Energie, die von dem ersten und zweiten Reflektor (22-3) reflektiert wurde;
als Reaktion, Erzeugen von Ionen an einer photokatalytischen Beschichtung auf der Mehrzahl an Durchlässen der ersten und zweiten Ziele (20); und
Führen eines Luftstroms durch die Mehrzahl an Durchlässen des ersten und zweiten Ziels (20), um die Ionen von dem ersten und zweiten Ziel (20) weg zu befördern, **gekennzeichnet durch**
Verhindern, unter Verwendung von UV-Reflektoren (22-1), dass ein emittierter UV-Strahl (26) durch die Ziele (20) hindurch verläuft.

10. Verfahren nach Anspruch 9, wobei der Schritt des Erzeugens von Ionen als Reaktion ferner ein Erzeugen von mehr Ionen an der photokatalytischen Beschichtung als Reaktion auf das Empfangen von UV-Energie von dem ersten und zweiten Reflektor (22-3) denn als Reaktion auf das Empfangen von UV-Energie direkt von dem UV-Emitter (24) aufweist.

11. Verfahren nach Anspruch 9, wobei der erste und der zweite Reflektor (22-3) ein Material aufweisen, das ein UV-Reflexionsvermögen von ungefähr 90% oder mehr bei UV-Wellenlängen von 185 nm und 254 nm aufweist.

## Revendications

1. Appareil d'ionisation d'air, l'appareil comprenant :
un émetteur d'UV (24) ;
une première paroi et une seconde paroi opposée à la première paroi ;
une première cible (20), la première cible (20) incluant :
une face interne disposée pour recevoir une énergie d'ultraviolets ("UV") de l'émetteur d'UV (24),
une face externe,
plusieurs passages entre la face interne et la face externe, et
un revêtement photo-catalytique sur les plusieurs passages ;
un premier réflecteur spéculaire (22-3) intercalé entre l'émetteur d'UV (24) et la première paroi ;
un second réflecteur spéculaire (22-3) intercalé entre l'émetteur d'UV (24) et la seconde paroi ;
**caractérisé par** :
une seconde cible (20) opposée à la première cible (20), la seconde cible (20) incluant :
une face interne disposée pour recevoir une énergie d'ultraviolets ("UV") de l'émetteur d'UV (24),
une face externe,
plusieurs passages entre la face interne et la face externe, et
un revêtement photo-catalytique sur les plusieurs passages ;
et
dans lequel les cibles (20) comprennent des réflecteurs d'UV (22-1) configurés pour éviter qu'un rayon UV émis (26) passe à travers les cibles (20).

2. Appareil selon la revendication 1, dans lequel une surface réfléchissante des premier et second réflecteurs (22-3) sont électriquement conductrices.

3. Appareil selon la revendication 1, dans lequel les premier et second réflecteurs (22-3) comprennent de l'acier inoxydable micro-poli.

4. Appareil selon la revendication 1, dans lequel les premier et second réflecteurs (22-3) comprennent un matériau qui ne s'oxyde pas facilement.

5. Appareil selon la revendication 1, dans lequel les premier et second réflecteurs (22-3) comprennent un matériau présentant une réflectivité d'UV d'environ 90 % ou supérieure aux longueurs d'onde UV de 185 nm et 254 nm.

6. Appareil selon la revendication 1, dans lequel la première cible (20) et/ou la seconde cible (20) comprend une matrice alvéolée.

7. Appareil selon la revendication 1, comprenant de plus :
un troisième réflecteur (22-2) placé dans un coin entre la première cible (20) et le premier réflecteur (22-3) ;
un quatrième réflecteur (22-2) placé dans un coin entre la seconde cible (20) et le premier réflecteur (22-3) ;
un cinquième réflecteur (22-2) placé dans un coin entre la première cible (20) et le second réflecteur (22-3) ; et
un sixième réflecteur (22-2) placé dans un coin entre la première cible (20) et le second réflecteur (22-3).

8. Appareil selon la revendication 1, comprenant de plus un septième réflecteur (22-1) et/ou un huitième réflecteur (22-1) sur la face interne de la première cible (20) ou seconde cible (20) et configuré pour réfléchir de l'énergie UV qui est émise à partir de l'émetteur d'UV dans une direction qui est presque orthogonale à la surface interne de la première cible (20) ou seconde cible (20), dans lequel le septième réflecteur et/ou le huitième réflecteur (22-1) est un réflecteur d'UV spéculaire.

9. Procédé d'ionisation d'air, le procédé comprenant :
l'émission, par un émetteur d'ultraviolets ("UV") (24), d'énergie UV vers un premier réflecteur (22-3), un second réflecteur (22-3) opposé au premier réflecteur (22-3), une première cible (20) et une seconde cible (20) opposée à la première cible (20) ;
la réflection, par le premier réflecteur (22-3) et le second réflecteur (22-3), d'énergie UV vers la première cible (20) et la seconde cible (20), dans lequel les premier et second réflecteurs sont des réflecteurs d'UV spéculaires ;
la réception, à plusieurs passages à travers les première et seconde cibles (20), d'énergie UV directement à partir de l'émetteur d'UV (24) et d'énergie UV réfléchie à partir des premier et second réflecteurs (22-3) ;
la production en réponse d'ions sur un revêtement photo-catalytique sur les plusieurs passages des première et seconde cibles (20) ; et
le passage d'un écoulement d'air à travers les plusieurs passages des première et seconde cibles (20) pour emporter les ions à distance des première et secondes cibles (20),
**caractérisé par**
la prévention qu'un rayon UV émis (26) passe à travers les cibles (20) par les réflecteurs d'UV (22-1).

10. Procédé selon la revendication 9, dans lequel l'étape de production en réponse d'ions comprend de plus la production en réponse de plus d'ions sur le revêtement photo-catalytique en réponse à la réception d'énergie UV des premier et second réflecteurs (22-3) qu'en réponse à la réception d'énergie UV directement à partir de l'émetteur d'UV (24).

11. Procédé selon la revendication 9, dans lequel les premier et second réflecteurs (22-3) comprennent un matériau ayant une réflectivité d'UV d'environ 90 % ou supérieure aux longueurs d'onde UV de 185 nm et 254 nm.
